# EUROPÄISCHE PATENTSCHRIFT

(11) **EP 1 682 658 B1**
(45) Veröffentlichungstag und Bekanntmachung des Hinweises auf die Patenterteilung: **26.12.2012**
(21) Anmeldenummer: 04797743.4
(22) Anmeldetag: 09.11.2004
(51) Int. Cl.: C12N 9/64, C12P 3/00, C07K 14/435, C01B 33/12, C08G 77/02, C08G 77/08

(54) **ENZYMATISCHE SYNTHESE, MODIFIKATION UND ABBAU VON SILICIUM (IV)- UND ANDERER METALL (IV)- VERBINDUNGEN**
ENZYMATIC SYNTHESIS, MODIFICATION AND DECOMPOSITION OF SILICON(IV) COMPOUNDS AND OTHER METAL(IV) COMPOUNDS
SYNTHESE, MODIFICATION ET DECOMPOSITION ENZYMATIQUES DE COMPOSES SILICIUM (IV) ET D'AUTRES COMPOSES METALLIQUES(IV)

(30) Priorität: 10.11.2003 DE 10352433
(43) Veröffentlichungstag der Anmeldung: 26.07.2006
(73) Patentinhaber: NanotecMARIN GmbH, 55128 Mainz (DE)
(72) Erfinder: MÜLLER, Werner, E., G., 65203 Wiesbaden (DE); SCHWERTNER, Heiko, 19055 Schwerin (DE); SCHRÖDER, Heinz, C., 65189 Wiesbaden (DE)
(74) Vertreter: Krauss, Jan
(86) Internationale Anmeldenummer: PCT/EP2004/012668
(87) Internationale Veröffentlichungsnummer: WO 2005/045020

(56) Entgegenhaltungen:
- WO-A-00/35993
- WO-A-02/10420
- DATABASE EMBL [Online] 2. Januar 2000 (2000-01-02), "Tethya aurantia silicatein beta mRNA, complete cds." XP002323543 gefunden im EBI accession no. EM_PRO:AF098670 Database accession no. AF098670
- DATABASE UniProt [Online] 5. Juli 2004 (2004-07-05), "Silicatein beta." XP002323488 gefunden im EBI accession no. UNIPROT:Q70TB1 Database accession no. Q70TB1
- MÜLLER WERNER E G ET AL: "Biochemistry and cell biology of silica formation in sponges." MICROSCOPY RESEARCH AND TECHNIQUE. 1 NOV 2003, Bd. 62, Nr. 4, 1. November 2003 (2003-11-01), Seiten 368-377, XP002323542 ISSN: 1059-910X

### SEQUENCE LISTING

<110> Johannes Gutenberg Universität Mainz und BioTec Marin GmbH
<120> Enzymatische Synthese, Modifikation und Abbau von Silicium(IV) - und anderer Metall (IV)-Verbindungen
<130> U30063PCT
<160> 2
<170> PatentIn version 3.2
<210> 1
   <211> 383
   <212> PRT
   <213> Suberites domuncula
<400> 1
   Met Ser Ala Leu Lys Phe Val Val Ala Leu Cys Val Val His Thr Ser
   1               5                   10                  15
   Leu Gly Ile Ala Glu Ser Val Gly Lys Ser Lys Thr Ala Gly Leu Ser
               20                  25                  30
   Asp Asp Gly Asn Tyr Thr Ala Val Thr Lys Ser Val Arg Leu Thr Pro
           35                  40                  45
   Val Leu Glu Phe Glu Glu Asp Trp Lys Gln Trp Thr Thr Asp His His
       50                  55                  60
   Lys Val Tyr Ser Asp Val Arg Glu Arg Val Asp Lys Tyr Thr Val Trp
   65                  70                  75                  80
   Arg Ala Asn Lys Glu Tyr Ile Asp Gln His Asn Gln Asn Ala Gln Arg
                   85                  90                  95
   Leu Gly Tyr Thr Leu Lys Met Asn Lys Phe Gly Asp Leu Thr Thr Lys
               100                 105                 110
   Glu Phe Ile Glu Gly Tyr His Cys Val Gln Asp Tyr Gln Pro Thr Asn
           115                 120                 125
   Ala Ser His Leu Asn Lys Lys His Lys Thr His Ala Phe Val Asp Tyr
       130                 135                 140
   Gly Asp Phe Val Arg Gly Gly Thr Gly Glu Gly Val Arg Gly Val Gly
   145                 150                 155                 160
   Asn Met Pro Glu Thr Met Asp Trp Arg Thr Ser Gly Val Val Thr Lys
                   165                 170                 175
   Val Lys Asp Gln Leu Arg Cys Gly Ser Ser Tyr Ala Phe Ser Ala Met
               180                 185                 190
   Ala Ser Leu Glu Gly Ile Asn Ala Leu Ser Tyr Gly Ser Leu Val Thr
           195                 200                 205
   Leu Ser Glu Gln Asn Ile Val Asp Cys Ser Val Thr Tyr Gly Asn His
       210                 215                 220
   Gly Cys Ala Cys Gly Asp Val Asn Arg Ala Leu Leu Tyr Val Ile Glu
   225                 230                 235                 240
   Asn Asp Gly Val Asp Thr Trp Lys Gly Tyr Pro Ser Gly Gly Asp Pro
                   245                 250                 255
   Tyr Arg Ser Lys Gln Tyr Ser Cys Lys Tyr Glu Arg Gln Tyr Arg Gly
               260                 265                 270
   Ala Ser Ala Arg Gly Ile Val Ser Leu Ala Ser Gly Asp Glu Asn Thr
           275                 280                 285
   Leu Leu Thr Ala Val Ala Asn Ser Gly Pro Val Ser Val Tyr Val Asp
       290                 295                 300
   Ala Thr Ser Thr Ser Phe Gln Phe Tyr Ser Asp Gly Val Leu Asn Val
   305                 310                 315                 320
   Pro Tyr Cys Ser Ser Ser Thr Leu Ser His Ala Leu Val Val Ile Gly
                   325                 330                 335
   Tyr Gly Lys Tyr Ser Gly Gln Asp Tyr Trp Leu Val Lys Asn Ser Trp
               340                 345                 350
   Gly Pro Asn Trp Gly Val Arg Gly Tyr Gly Lys Leu Ala Arg Asn Lys
           355                 360                 365
   Gly Asn Lys Cys Gly Ile Ala Thr Ala Ala Ser Phe Pro Thr Leu
       370                 375                 380
<210> 2
   <211> 1372
   <212> DNA
   <213> Suberites domuncula
<400> 2
   acttagtata ttatggtagt gtacaatacc tatctccata gaggcatgca taactaggtt     60
   tattgaatag ctgctggcac aatttgttct caagttggtg ctattagatt tgtgttctag    120
   aatgtcagca ttgaagtttg tagttgcctt gtgtgtagtt cacacaagct taggaatagc    180
   tgagtcagtt ggtaagagca agactgcagg cctaagtgac gatggcaact acacagctgt    240
   caccaaatct gtaagactga ctccagttct agagtttgag gaagattgga agcaatggac    300
   aactgatcat cacaaggtct actctgatgt gagggagaga gtggacaagt acactgtatg    360
   gagagctaat aaagagtaca ttgatcaaca caaccagaac gcacagagat tgggatacac    420
   actcaaaatg aacaaatttg gagatttgac taccaaggag ttcattgaag gctatcactg    480
   tgttcaggac taccaaccta ccaatgcatc acatttgaat aagaaacaca aaacgcacgc    540
   gtttgtcgac tatggtgact ttgtgagggg tggaactggt gagggtgtga ggggtgtagg    600
   aaacatgccg gagactatgg actggagaac ttctggagtt gtcacaaaag ttaaagatca    660
   gcttcgttgt ggtagcagct atgcgttctc tgccatggct tcattggaag gaataaatgc    720
   tctttcctac ggatctttgg tgacactcag tgaacaaaac attgtagact gctcggttac    780
   ctatggcaac catggttgcg cctgtggtga tgtaaaccgt gctctactgt atgtgataga    840
   gaatgatggc gttgacacgt ggaagggtta tccttctggt ggggatcctt atcgatcaaa    900
   gcaatactct tgcaaatacg agagacagta tcgtggggcc tctgctagag gtatagtcag    960
   tctagctagt ggtgatgaga acacattgtt gacagcagta gctaactctg gaccagtgag   1020
   tgtgtatgtg gacgctactt caacatcctt ccagttttac agtgatggag tgttgaatgt   1080
   tccctattgc tcctctagca cgctgagtca tgccttggtt gtcattggtt acgggaagta   1140
   cagcggacaa gattactggc ttgttaaaaa cagctggggt cctaactggg gagtgcgggg   1200
   ctatgggaag ttggcaagaa acaagggcaa caaatgtgga atagccacag cggctagttt   1260
   cccaacatta tgacacttta gttgatcaaa caattaatca taaattatta caacatgtag   1320
   tataatgatg cccccccatt gctcaatagc ttatctttga acaagaaaaa aa           1372

## Beschreibung

Die vorliegende Erfindung betrifft ein Verfahren zur *in vitro* synthese oder *in vivo* Synthese in einer isolierten transfizierten wirtszdle von Siliciumdioxid, Siliconen und anderen Silicium(IV)- oder Metall(IV)-Verbindungen sowie von Mischpolymeren dieser Verbindungen, wobei ein Polypeptid gemäß SEQ ID Nr. 1 oder ein Metallkomplex dieses Polypeptids zur Synthese eingesetzt wird. Die vorliegende Erfindung betrifft auch ein Polypeptid eines Silicatein aus *Suberites domuncula* gemäß SEQ ID Nr. 1, order ein Metallkomplex des Polypeptids.

### 1. Stand der Technik

Siliciumverbindungen wie Silicate und Silicone (Siloxane) sind vielbenutzte Materialien in Industrie und Medizin und auch in wirtschaftlicher Hinsicht bedeutsam. Viele Hochtechnologie-Produkte wie optische und mikroelektronische Instrumente sowie Katalysatoren enthalten oder bestehen aus Silicaten oder Silicon-Verbindungen.

Das tetraedrisch gebaute Silicat-Anion ([SiO₄]⁴⁻; Monomer) neigt zur Polymerisation durch Verknüpfung von SiO₄-Einheiten, wobei jeweils zwei Si-Atome über ein O-atom miteinander verbunden werden. Durch Wasserabspaltung (Kondensation) entsteht dabei aus der Orthokieselsäure zunächst die Orthodikieselsäure (Pyrokieselsäure; H₆Si₂O₇). Die weitere Kondensation führt über die Polykieselsäuren zu den Metakieselsäuren [(H₂SiO₃)ₙ]. Bei kleinerer Zahl der SiO₄-Einheiten (n = 3, 4 oder 6) können sich dabei auch ringförmige Moleküle bilden. Bei weiterem Fortschreiten der Kondensation entstehen aus den zunächst erhaltenen Ketten oder Netzen dreidimensionale Strukturen, welche der Zusammensetzung SiO₂ entsprechen (CD Römpp Chemie Lexikon - Version 1.0, Stuttgart/New York: Georg Thieme Verlag 1995).

Das (polymere) Siliciumdioxid (SiO₂) kommt sowohl in kristalliner als auch in amorpher Form vor. Zu den verschiedenen Formen des kristallinen SiO₂ gehören u. a. Quarz, Tridymit und Cristobalit. Amorphe Siliciumdioxid-Mineralien sind u.a. Achat, Opal und Feuerstein. Aus amorphem SiO₂ (auch Silica genannt) bestehen auch viele, durch Biomineralisation entstandene Skelettstrukturen, wie die Schalen von Kieselalgen (Diatomeen) und die Nadeln (Spiculae) von Kieselschwämmen. In allen diesen SiO₂-Formen besitzt Silicium die Koordinationszahl 4 und ist tetraedrisch von vier Sauerstoff-Atomen umgeben.

Durch teilweises Ersetzen der OH-Gruppen der Kieselsäure durch einbindige Organylreste, die sich nicht am Kondensationsvorgang beteiligen, entstehen verschiedene Silicone (Siloxane). Sie werden eingeteilt in lineare, verzweigte und cyclische Polysiloxane sowie vernetzte Polymere (Verknüpfung von ketten- oder ringförmige Molekülen zu zwei- oder dreidimensionalen Netzwerken).

Die Viskosität der kettenförmig aufgebauten Silicone (Siliconöle) nimmt mit wachsender Kettenlänge zu. Silicone, die in geringem Maß vernetzt sind, zeigen Kautschuk-Elastizität (Siliconkautschuk), stark vernetzte Ketten sind harzartig (Siliconharze).

Die vorliegende Erfindung betrifft ein Verfahren zur *in vitro* Synthese order *in vivo* Synthese in einer isolierten transtizieston Wirtszelle von Siliciumdioxid, Siliconen und anderen Silicium(IV)- oder Metall(IV)-Verbindungen sowie von Mischpolymeren dieser Verbindungen, wobei ein Polypeptid gemäß SEQ ID Nr. 1 oder ein Metallkomplex dieses Polypeptids zur Synthese eingesetzt wird.

Die vorliegende Erfindung betrifft auch ein Polypeptid eines Silicatein aus *Suberites domuncula* gemäß SEQ ID Nr. 1 order ein Metallkomplex des Polypeptids.

Weitere vorteilhafte Ausführungsformen der Erfindung sind in den beigefügten Ansprüchen beansprucht.

### 1.1 Biomineralisation (Bildung von biogenem Siliciumdioxid) in Kieselschwämmen

Die technische Synthese der Silicate erfordert drastische Bedingungen wie hoher Druck und hohe Temperatur. Kieselschwämme sind dagegen - ebenso wie Kieselalgen - befähigt, mit Hilfe spezifischer Enzyme Silicatgerüste unter milden Bedingungen, d. h. bei relativ niedriger Temperatur und niedrigem Druck, zu bilden.

Die Hauptelemente des Skeletts der Kieselschwämme sind die nadelförmigen Spiculae, die bei der Gruppe der Demospongien (Hornschwämme) und Hexactinellida (Glasschwämme) aus amorphem nichtkristallinem Siliciumdioxid bestehen.

Der Stand des Wissens über die Morphologie und Biogenese der Spiculae ist dargestellt in: Uriz et al. (2003) Progr Molec Subcell Biol 33:163-193; Müller et al. (2003) Progr Molec Subcell Biol 33:195-221. Das opale Siliciumdioxid in Schwamm-Spiculae enthält 6-13% Wasser, was die ungefähre Formel (SiO₂)₂₋₅•H₂O ergibt (Schwab & Shore (1971) Nature 232:501-502). In Demospongien beginnt die Spiculaebildung um ein axiales Filament, um das Silica enzymatisch abgelagert wird.

Zwei Enzyme, die bei silicatbildenden Organismen an der Synthese und/oder dem Abbau des SiO₂-Skeletts beteiligt sind, und ihre technische Verwendung wurden beschrieben.

Bei dem einen Enzym handelt es sich um Silicatein-α (auch lediglich Silicatein genannt), bei dem es sich um das Protein handelt, welches das den Axialkanal der Schwamm-Spiculae (Nadeln) ausfüllende Axialfilament aufbaut WO00/35993. Methods, compositions, and biomimetic catalysts, such as silicateins and block copolypeptides, used to catalyze and spatially direct the polycondensation of silicon alkoxides, metal alkoxides, and their organic conjugates to make silica, polysiloxanes, polymetallo-oxanes, and mixed poly(silicon/ metallo)oxane materials under environmentally benign conditions. Inventors/ Applicants: Morse DE, Stucky GD, Deming, TD, Cha J, Shimizu K, Zhou Y; DE 10037270 A1. Silicatein-vermittelte Synthese von amorphen Silicaten und Siloxanen und ihre Verwendung. Deutsches Patentamt 2000. Anmelder und Erfinder: Müller WEG, Lorenz B, Krasko A, Schröder HC; WO 02/10420. Silicatein-mediated synthesis of amorphous silicates and siloxanes and use thereof. Inventors/Applicants: Müller WEG, Lorenz B, Krasko A, Schröder HC). Dieses Enzym wurde aus dem marinen Kieselschwamm *Suberites domuncula* kloniert (Krasko A, Batel R, Schröder HC, Müller IM, Müller WEG (2000) Expression of silicatein and collagen genes in the marine sponge S. domuncula is controlled by silicate and myotrophin. Europ J Biochem 267:4878-4887). Silicatein ist in der Lage, aus organischen Siliciumverbindungen (Alkoxysilanen) amorphes Siliciumdioxid (Polykieselsäuren und Polysilicate) zu synthetisieren (Cha JN, Shimizu K, Zhou Y, Christianssen SC, Chmelka BF, Stucky GD, Morse DE (1999) Silicatein filaments and subunits from a marine sponge direct the polymerization of silica and silicones in vitro. Proc Natl Acad Sci USA 96:361-365).

Das zweite Enzym ist die Silicase, die zur Gruppe der Carboanhydrasen gehört (DE 102 46 186.4**.** Abbau und Modifizierung von Silicaten und Siliconen durch Silicase und Verwendung des reversiblen Enzyms. Deutsches Patentamt 2002. Anmelder und Erfinder: Müller WEG, Krasko A, Schröder HC). Dieses Enzym, das zuerst im Meeresschwamm *S*. *domuncula* entdeckt wurde, ist primär am Abbau von biogenem Silica beteiligt (Schröder HC, Krasko A, Le Pennec G, Adell T, Wiens M, Hassanein H, Müller IM, Müller WEG (2003) Silicase, an enzyme which degrades biogenous amorphous silica: Contribution to the metabolism of silica deposition in the demosponge Suberites domuncula. Progr Molec Subcell Biol, 33, 250-268), aber auch in der reversiblen Reaktion zu deren Synthese in der Lage.

WO 02/10420 A beschreibt Silicatein-alpha und Verwendungen zur Synthese von Siliziumverbindungen. WO 00/35993 offenbart ebenfalls Silicateine und deren Verwendungen zur Synthese von Siliziumverbindungen. Die Database Accession Number AF098670 offenbart die Tethya aurantia silicatein beta mRNA.

### 2. Gegenstand der Erfindung

Überraschenderweise wurde von den Erfindern - zunächst im Meeresschwamm *S*. *domuncula* - entdeckt, daß in Zellen von Silica-bildenden Organismen nicht nur ein Silicasynthetisierendes Enzym (Silicatein-α), sondern weitere Silica-bildende Enzyme vorkommen. Das hier beschriebene Silicatein-β zeichnet sich hierbei durch besonders vorteilhafte Eigenschaften im Hinblick auf seine katalytischen Fähigkeiten und deren technische/medizinische Anwendbarkeit aus, die nicht aus dem Stand der Technik und Analogiefolgerungen für den Fachmann zu erkennen sind.

Diese vorteilhaften Eigenschaften beruhen auf:
a) unterschiedlicher Substratspezifität
b) unterschiedlicher Kinetik
c) unterschiedlichen Bindekonstanten
d) unterschiedlicher Stabilität.

Die für das Silicatein-β-Polypeptid codierende cDNA wurde mit Hilfe der PCR-Methode aus *S. domuncula* isoliert (siehe unten). Verwandte cDNAs können auch aus weiteren Schwämmen, wie z.B. *Geodia cydonium,* isoliert werden.

Neu an der vorliegenden Erfindung ist außerdem, daß das Silicatein-β-Gen in Tieren (Schwämmen) oder daraus gewonnenen Zellen/Zellaggregaten durch Erhöhung der Silicium-Konzentrationen im Medium (gewöhnlicherweise auf 60 µM Silicat oder eine andere Siliciumverbindung, bei deren Hydrolyse eine ebenso große Konzentration an Silicat erreicht wird) induziert werden kann. Besonders vorteilhaft ist (eine noch stärkere Induktion wird erreicht), wenn neben Silicium auch eine erhöhte Eisenkonzentrationen (Eisenionen bzw. Eisensalze oder Komplexe wie Fe(+++) Citrat) im Medium vorliegt.

Gemäß eines ersten Aspekts der vorliegenden Erfindung wird allgemein ein Verfahren zur *in vitro* Synthese order *in vivo* Synthese in einer isolierten transfizierten wirtszelle von Siliciumdioxid, Siliconen und anderen Silicium(IV)- oder Metall(IV)-Verbindungen sowie von Mischpolymeren dieser Verbindungen, wobei ein Polypeptid gemäß SEQ ID Nr. 1 oder ein Metallkomplex dieses Polypeptids zur Synthese eingesetzt wird. Bisher war nicht bekannt und nicht aus dem Stand der Technik zu erkennen, daß neben Silicatein-α weitere Enzyme, wie das hier beschriebene Silcatein-β, in der Lage sind, Silicate oder Silicone aufzubauen. Aufgrund der Reversibilität dieses Prozesses betrifft ein weiterer Aspekt der vorliegenden Erfindung ein Verfahren zum Abbau von amorphem Siliciumdioxid, Siliconen und anderen Silicium(IV)- oder Metall(IV)-Verbindungen sowie von Mischpolymeren dieser Verbindungen, wobei ein Polypeptid gemäß SEQ ID Nr. 1 oder ein Metallkomplex dieses Polypeptids zum Abbau eingesetzt wird.

Es wird ein erfindungsgemäßes Verfahren eingesetzt, das dadurch gekennzeichnet ist, daß zur Synthese Verbindungen wie Kieselsäuren, Monoalkoxysilantriole, Monoalkoxysilandiole, Monoalkoxysilanole, Dialkoxysilandiole, Dialkoxysilanole, Trialkoxysilanole, Tetraalkoxysilane, Alkyl-, Aryl- oder Metallo-Silantriole, Alkyl-, Aryl- oder Metallo-Silandiole, Alkyl-, Aryl- oder Metallo-Silanole, Alkyl-, Aryl- oder Metallo-Monoalkoxysilandiole, Alkyl-, Aryl - oder Metallo-Monoalkoxysilanole, Alkyl-, Aryl- oder Metallo-Dialkoxysilanole, Alkyl-, Aryl- oder Metallo-Trialkoxysilane oder andere Metall(IV)-Verbindungen als Reaktanten (Substrate) eingesetzt werden. Durch Verwendung definierter Mischungen der Verbindungen, wobei das Verhältnis der Substrate frei gewählt werden kann, können Mischpolymere definierter Zusammensetzung hergestellt werden.

Gemäß einem weiteren Aspekt der vorliegenden Erfindung wird ein Verfahren zur Modifikation einer Kieselsäure oder Silicium(IV)- oder Metall(IV)-Verbindung enthaltenden Struktur oder Oberfläche zur Verfügung gestellt, wobei ein Polypeptid gemäß SEQ ID Nr. 1 oder ein Metallkomplex dieses Polypeptids zur Modifikation eingesetzt wird. Bevorzugterweise liegt die Kieselsäure enthaltende Struktur oder Oberfläche in Form eines Edelsteins oder Halbedelsteins vor.

Es wird ein erfindungsgemäßes Verfahren eingesetzt, wobei die Modifikation vergleichbar einer Glättung, eines Anätzens oder ein Herstellen von Ausbohrungen bzw. Aussparungen der Kieselsäure oder Silicium(IV)- oder Metall(IV)-Verbindung enthaltenden Struktur oder Oberfläche durch das Polypeptid oder einen Metallkomplex des Polypeptids umfaßt.

Ein weiterer Aspekt der vorliegenden Erfindung betrifft auch ein Polypeptid eines Silicatein-β aus *Suberites domuncula* gemäß SEQ ID Nr. 1, order ein Metallkomplex des Polypeptids.

Ein weiterer Aspekt der vorliegenden Erfindung betrifft auch eine Nukleinsäure, insbesondere gemäß SEQ ID Nr. 2, dadurch gekennzeichnet, daß sie für ein Polypeptid der Erfindung kodiert. Die erfindungsgemäße Nukleinsäure kann in Form einer DNA, cDNA, RNA oder Gemischs davon vorliegen und dadurch gekennzeichnet sein, daß die Sequenz der Nukleinsäure mindestens ein Intron und/oder eine polyA-Sequenz aufweist. Ein anderer Aspekt der vorliegenden Erfindung betrifft die erfindungsgemäße Nukleinsäure in Form ihrer komplementären "antisense"-Sequenz.

Ein noch weiterer Aspekt der vorliegenden Erfindung betrifft auch eine erfindungsgemäße Nukleinsäure in Form eines (a) Fusionsprotein- (chimäres Protein) Konstrukts, (b) Konstrukts mit getrennter Protein-Expression (Protease-Spaltstelle) oder (c) Konstrukts mit getrennter Protein-Expression (Kassetten-Expression). Die erfindungsgemäße Nukleinsäure kann synthetisch hergestellt worden sein. Verfahren dazu sind im Stand der Technik gut bekannt.

Das erfindungsgemäße Polypeptid kann, neben der natürlichen Form, ferner dadurch gekennzeichnet sein, daß es synthetisch hergestellt worden ist oder daß das Polypeptid oder der Metallkomplex des Polypeptids in einem prokaryotischen oder eukaryotischen Zellextrakt oder - lysat vorliegt. Der Zellextrakt oder das Lysat kann aus einer Zelle *ex vivo* oder *ex vitro* gewonnen werden, zum Beispiel einer rekombinanten bakteriellen Zelle oder einem Meeresschwamm.

Das erfindungsgemäße Polypeptid kann nach herkömmlichen im Stand der Technik bekannten Verfahren gereinigt werden und somit im wesentlichen frei von anderen Proteinen vorliegen.

Ein weiterer Aspekt der vorliegenden Erfindung betrifft dann ein Verfahren zur Auffindung von Inhibitoren oder Aktivatoren eines Polypeptids des Silicatein-β aus *Suberites domuncula* gemäß SEQ ID Nr. 1, wobei a) ein Polypeptid des Silicatein-β aus *Suberites domuncula* gemäß SEQ ID Nr. 1 zur Verfügung gestellt wird, b) das Polypeptid aus Schritt a) mit einem potentiellen Inhibitor oder Aktivator in Kontakt gebracht wird, und c) die Fähigkeit des Polypeptids gemessen wird, Silicate oder Silicone zu synthetisieren oder abzubauen. Mit diesem Verfahren lassen sich wertvolle Substanzen aufspüren, die sich unter Umständen als Therapeutika eignen (dazu siehe im folgenden). Verfahren zur Auffindung solcher Substanzen sind dem Fachmann bekannt und schließen z.B. die Verwendung von radioaktiv markierten oder enzymatisch markierten Kandidaten-Verbindungen ein. Verfahren zur Messung der Aktivität der Hydrolase (Silicatein-β) sind im folgenden beschrieben und können leicht durch den Fachmann auf ein Testformat hin angepaßt werden. Ein Inhibitor verringert dabei die Aktivität des Enzyms im wesentlichen vollständig, ein Aktivator induziert eine Aktivität oder verstärkt diese über den Ausgangsspiegel.

Gemäß einer Alternative des Verfahrens kann das Polypeptid einer Hydrolase (Silicatein-β) aus *Suberites domuncula* gemäß SEQ ID Nr. 1 in einem prokaryontischen oder eukaryontischen Zellextrakt oder -lysat oder in gereinigter Form für den Test zur Verfügung gestellt wird.

Die Erfindung soll nun in den folgenden Beispielen weiter verdeutlicht werden, ohne darauf beschränkt zu werden. In den beigefügten Figuren und dem Sequenzprotokoll zeigen:
Im nachfolgenden sind die erläuternden Legenden zu den beigefügten Abbildungen und den Sequenzprotokollen aufgeführt. Es zeigen:
   **SEQ ID Nr. 1:** Die Aminosäuresequenz des erfindungsgemäßen Silica-metabolen Silicatein-β-Polypeptids aus *S. domuncula* (rSILICAß_SUBDO),
   **SEQ ID Nr. 2**: Die Nukleinsäuresequenz der cDNA des erfindungsgemäßen Silica-metabolen Silicatein-β-Polypeptids aus *S*. *domuncula.*
   **Figur 1****:**
      Die Nukleinsäuresequenz der cDNA des erfindungsgemäßen Silica-metabolen Silicatein-β-Polypeptids aus *S. domuncula.* Die aus der Nukleotidsequenz des offenen Leserahmens abgeleitete Aminosäuresequenz ist unterhalb der Nukleotidsequenz angegeben.
   **Figur 2****:**
      Gezeigt ist ein Vergleich ("Alignment") der Schwamm-Silicatein-Sequenzen, Silicatein-α und Silicatein-(3, von *S*. *domuncula* (SILICAaSUBDO [Datenbank-Eintragungsnummer CAC03737.1] und SILICAbSUBDO [AJ519940]) und *T. aurantium* (SILICAaTETHYA [AAC23951.1]; SILICAbTETHYA [AF098670]). Konservierte Reste (ähnlich oder verwandt imHinblick auf ihre physikalisch-chemischen Eigenschaften) in den Sequenzen werden in weiß auf schwarz gezeigt und solche in mindestens zwei Sequenzen in schwarz auf grau. Die charakteristischen Stellen innerhalb der Silicatein-Sequenzen sind angegeben: Ser (●), His (■) und Asn (■), die Prozessierungsstelle für die Umwandlung des Proenzyms zum reifen Enzym ist markiert (} {), ebenso das Serin-Cluster ([ Ser ]).
   **Figur 3****:**
      Phylogenetische Beziehungen des Schwamm-Silicateins mit den Cathepsin L-Sequenzen von Protostomiern (*D. melanogaster* [DROSOPHILA] BAA06738; *Caenorhabditis elegans* [CAENORHABDITIS] NP_507199; *Artemia franciscana* [ARTEMIA] AAD39513) und Deuterostomiern (*Danio rerio* [DANIO] AAN32912.1; *Homo sapiens* [HUMAN] X12451) zusammen mit den Schwammsequenzen von *S*. *domuncula* ([SUBERITES]; AJ272013) und *G. cydonium* ([GEODIA]; Y10527). Der Baum blieb "ungerooteter" [ohne Außengruppe "root"], um das Clustering der Cathepsine und Silicateine zu zeigen. Die Zahlen an den Verzweigungen geben die statistische Signifikanz der Verzweigungen an (1000 entspricht 100% Signifikanz). Die Skala gibt die sogenannte "evolutionäre Distanz" wieder, wobei die Länge der Skala einer Distanz von 0.1 Aminosäure-Substitutionen pro Position innerhalb der Sequenz entspricht.
   **Figur 4****:**
      Höhe der Expression von *silicatein* in Gewebe von *S*. *domuncula* nach Überführung in SilicatlFe(+++)-supplementiertes künstliches Seewasser. Die RNA wurde entweder sofort nach der Zugabe von Silicat/Fe(+++) (Tag Null) oder zwei und sechs Tage später extrahiert. Northem-Blot-Analyse.
   **Figur 5****:**
      Nachweis von *silicatein*-positiven Zellen in Schwammgewebe. (**A**) Cryoschnitte wurden von einem Schwamm hergestellt, der in Silicat/Fe(+++)-freiem Seewasser gehalten wurde, und mit DIG-markierter *SDSILICA*β-antisense DNA hybridisiert. Anschließend wurden die Proben mit Anti-Digoxigenin/alkalischer Phosphatase inkubiert und die Signale mit NBT/X-Phosphat detektiert. Analyse der Schnitte von Tieren, die für 2 (**B**), 4 (**C**) oder 6 Tage (**D**) in Silicat/Fe(+++)-supplementiertem Seewasser gehalten wurden. Die Kanäle (c) des wasserhaltigen. Systems innerhalb des Mesohyl (m) sind gezeigt. Die Kanäle werden von einer epithelialen Schicht begrenzt, die von Pinacocyten gebildet wird. Vergrößerung: x 50.

### Klonierung des das Silicatein-β kodierenden Gens

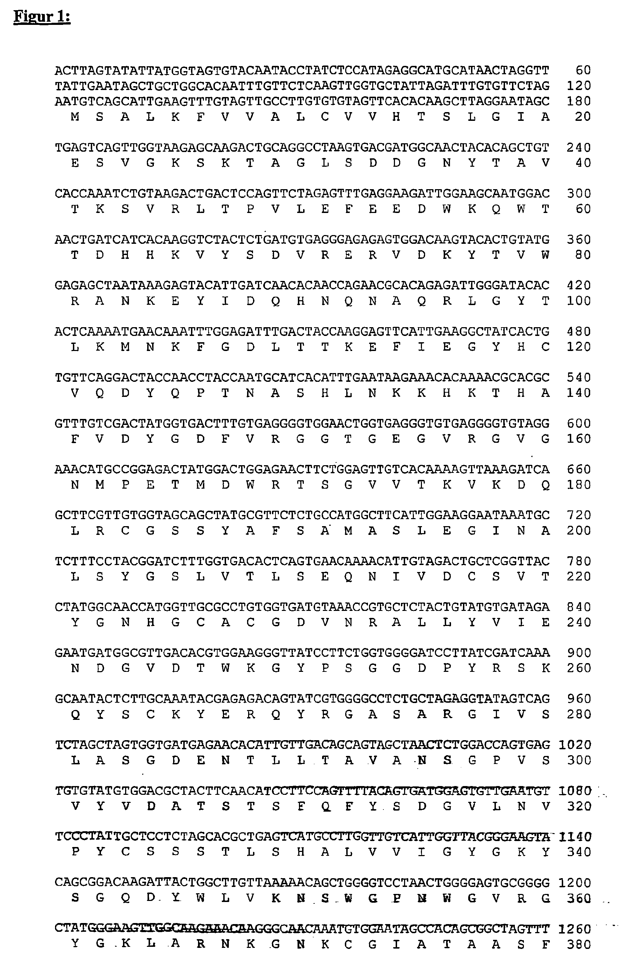

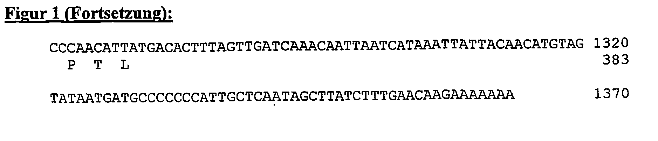

Eine cDNA, die für das Silicatein-β von S*. domuncula* kodiert, wurde ans einer cDNA Bibliothek isoliert (Abbildung 1). Bei Silicatein-β war bisher lediglich bekannt, daß es als Proteinkomponente des axialen Filaments der Spiculae (Schwamm-Nadeln) vorkommt, nicht jedoch eine Beteiligung an der enzymatischen Bildung von Siliciumdioxid-Spiculae.

Die Isolierung der cDNA für Silicatein-β z.B. aus *S*. *domuncula* wird folgendermaßen durchgeführt. Zum Homologie-Screening wird eine Digoxigenin-11-dUTP-markierte DNA-Sonde ("DIG random primed DNA labelling kit"; Fa. Roche) von *Silicatein-α* aus S *domuncula* benutzt; die Sequenz dieser cDNA ist beschrieben (Datenbank-Eintragungsnummer AJ272013; Krasko et al. (2000) Europ J Biochem 267:4878-4887). Das Screening der *S. domuncula*cDNA-Bibliothek wird unter "low stringency"-Hybridisierungs-Bedingungen der "plaque lifts" wie beschrieben (Kruse et al. (1997) Mol Biol Evol 14:1326-1334) durchgeführt. Positive Klone werden mit einem alkalische Phosphatase-konjugiertem Anti-Digoxigenin-Antikörper unter Benutzung von BCIP/NBT als Substrat identifiziert (Blake et al. (1984) Anal Biochem 136:75-179). Durch Northern-Blotting kann bestimmt werden, ob die komplette Sequenz *SDSILICAβ* erhalten wurde. Die DNA-Sequenzierung kann mit einem automatischen DNA-Sequencer durchgeführt (Li-Cor 4000S) werden.

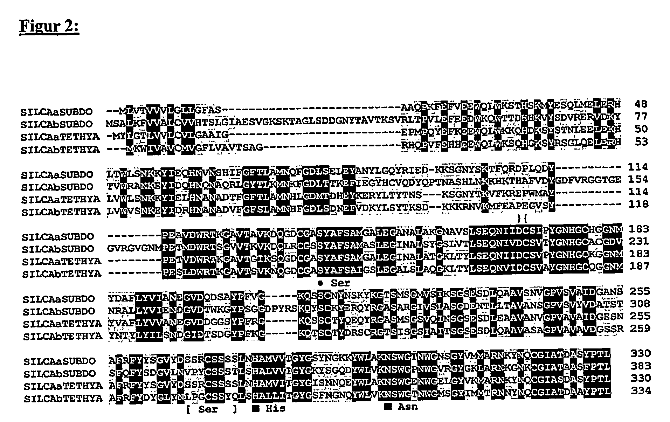

Die für das Silicatein-β-Polypeptid aus dem Meeresschwamm S. *domuncula* codierende cDNA sowie das aus der Nucleotidsequenz abgeleitete Polypeptid besitzen folgende Eigenschaften. Die Nucleotidsequenz umfaßt 1372 Reste mit einem offenen Leserahmen von nt₁₂₂-₁₂₄ bis nt₁₂₇₁₋₁₂₇₃ (Stoppcodon) (Abbildung 1). Die abgeleitete Aminosäuresequenz des Silicatein-β stellt mit 383 Resten ein 42,068 Da großes Polypeptid dar (Abbildung 1 und Abbildung 2).

Die Schwamm-Silicatein-β-Polypeptid ist ein neues Mitglied der Cathepsin L Unterfamilie (Shimizu et al. (1998) Proc Natl Acad Sci USA 95:6234-6238; Cha et al. (1999) Proc Natl Acad Sci USA 96:361-365; Krasko et al. (2000) Europ J Biochem 267:4878-4887). Das *S*. *domuncula*-Silicatein-β-Polypeptid besitzt die größte Ähnlichkeit zu Silicatein-α von *Tethya aurantium* (Datenbank-Eintragungsnummer AAC23951.I; "Expect value" [*E*; Blast-NCBI; (Coligan et al. (2000) Current Protocols in Protein. Science John Wiley & Sons, Chichester)] = 7e⁻⁵⁸), zu Silicatein-β von *T. aurantium* (AF098670; *E* = 4e⁻⁵⁷) und zu Silicatein-α von *S*. *domuncula* (CAC03737.1; *E* = 2e⁻⁵⁶); etwas entfernter verwandt ist das *S*. *domuncula* Silicatein-β zu Cathepsin L von *Rhodnius prolixus* (AF320565; E = 3e⁻⁵⁵)- Alle vier Silicateinse-quenzen zeigen die charakteristichern drei Aminosäuren Ser (anstelle von Cys, das in den Cathepsinen worhanden ist), His und Asn, welche die katalytische Triade der Cystein-Proteasen bilden (Shimizu et al. (1998) Proc Natl Acad Sci USA 95:6234-6238; Cha et al. (1999) Proc Natl Acad Sci USA 96:361-365; Krasko et al. (2000) Europ J Biochem 267:4878-4887); Abbildung 2. Die Pozessierungsstelle, die für die Bildung der aktiven Enzyme erforderlich ist, entweder als Ergebnis einer Autolyse oder durch eine zweite Protease, kann nach: Nishimura et al. ((1988) Arch Biochem Biophys 261:64-71) vorhergesagt werden. Auf Grund von Vergleichen mit den Cathepsinen kann die Spaltstelle in dem Schwamm-Silicatein-β bei aa₁₃₉ lokalisiert werden (Abbildung 2); daher wird das Proenzym mit Mᵣ 42,068 Da zu einem aktiven Enzym mit einem vorhergesagten Mᵣ von 26,205 Da reifen. Die drei mutmaßlichen Disulfidbindungen sind in dem Schwamm-Silicatein-β ebenso wie in den anderen Cystein-Proteasen vorhanden.

### Phylogenetische Analyse des Silicatein-β-Polypeptids

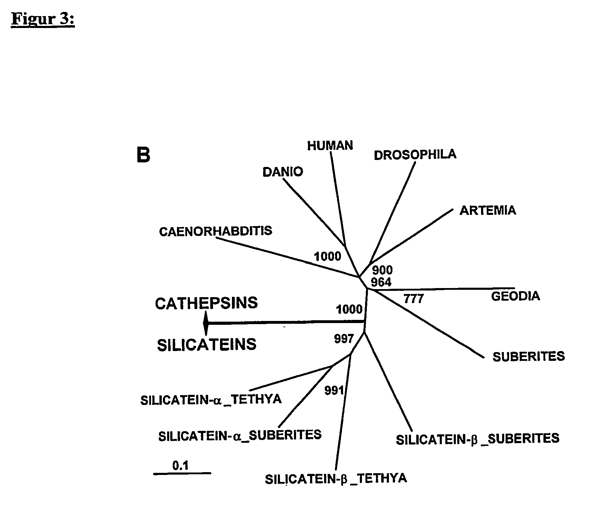

Für die phylogenetische Analyse (Abbildung 3) wurde ein Vergleich ("Alignment") der Schwamm-Silicateine von *S*. *domuncula* (Silicatein-α und Silicatein-β) und *T. aurantium* (Silicatein-α und Silicatein-β) mit den Cathepsin L-Sequenzen von Protostomiern *(Drosophila melanogaster, Caenorhabditis elegans* und *Artemia franciscana)* und Deuterostomiern (*Homo sapiens* und *Danio rerio*) zusammen mit den Cathepsin-Sequenzen von *S*. *domuncula* und *G*. *cydonium* durchgeführt. Der "ungerootete" [ohne Außengruppe "root"] Baum zeigt, daß die Cathepsin L-Sequenzen von den vier Silicateinen klar getrennt sind.

### Hochregulation des Silicatein-β-Polpeptids

Die Expression des *silicatein-β-Gens* läßt sich durch Zusatz von Silicat und Fe(+++) im Medium hochregulieren. Dies läßt sich sowohl beispielsweise durch Northem-Blotting oder Western-Blotting an ganzen Tieren, Geweben, Zellen oder Zellaggregaten (wie Schwamm-Primmorphen) zeigen.

Die letztgenannnten Primmorphe sind aggreate, die ans proliferierenden und differenzieren den Zellen bestehen und aus Schwamm-Einzelzellen gebildet werden. Das Primmorphe-System wurde zum Patent angemeldet (DE 19824384. Herstellung von Primmorphe aus dissöziierten Zellen von chwämmen, Korallen und weiteren Invertelraten Verfahren zur Kultivierung von Zellen von Schwämmen und weiteren Invertebraten zur Produktion und Detektion von bioaktiven Substanzen, zur Detektion von Umweltgiften und zur Kultivierung dieser Tiere in Aquarien und im Freiland. Erfinder und Anmelder: Müller WEG, Brümmer F).

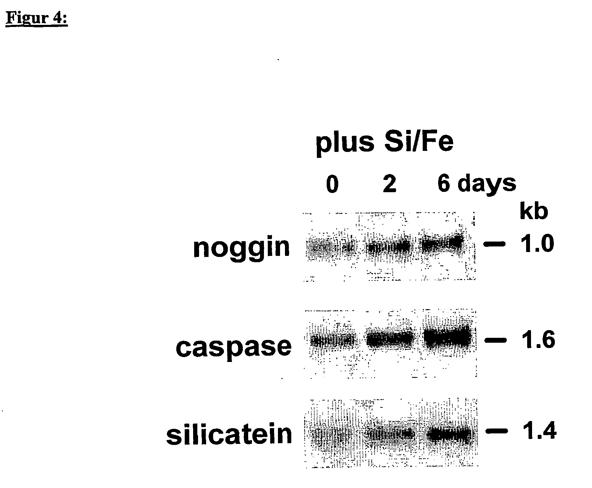

In dem in Abbildung 4 gezeigten Experiment wurde die Höhe der Expression des *silicatein*-β-Gens bestimmt, die als Antwort auf Silicat und Fe(+++) im Medium stark hochreguliert wird.

Zur Bestimmung des Effektes von Silicium und Eisen auf die Genexpression wurden die Tiere für zwei Wochen in künstlichem Seewasser kultiviert, das zusammengesetzt ist aus Chlorid (19.0 g/kg), Natrium (11.0), Magnesium (1.3), Sulfat (2.7), Calcium (0.4), Kalium (0.4) und Bicarbonat (0.15). Dann wurden die Tiere in künstliches Seewasser, ergänzt mit 60 µM Silicat (als Na-Silicat) und 30 µM Fe(+++), überführt.

### Northern-Blotting

Die RNA wird aus in flüssigem Stickstoff pulverisierten Tieren, Geweben, Zellen oder Zellaggregaten mit TRIzol-Reagens extrahiert (fa. GibcoBRL). Dann wird eine Menge von 5 µg Gesamt-RNA elektrophoretisch an einem 1%igen Formaldehyd/Agarose-Gel aufgetrennt und auf eine Hybond-N⁺-Nylon-Membran geblottet entsprechend den Instruktionen des Herstellers (Fa. Amersham). Die Hybridisierung wird mit der cDNA *SDSILICA*β durchgeführt. Verwendet werden kann beispielsweise eine *silicatein-*β*-Sonde,* die von nt₆₇₆ bis nt₁₁₉₈ reicht und somit die charakteristische Region in dem abgeleiteten Polypeptid, nämlich das Serin-reiche Cluster, überspannt. Dieses Cluster wird im S. *domuncula* Silicatein-β-Polypeptid zwischen aa₃₀₉ bis aa₃₂₉ gefunden; diese Region zeigt auch Unterschiede zu dem Silicatein-α-Polypeptid. Als interner Standard kann beispielsweise die *S*. *domuncula* β-Tubulin cDNA, *SDTUB* (Datenbank-Eintragungsnummer AJ550806) benutzt werden. Die Sonden werden mit dem PCR-DIG-Probe-Synthesis Kit entsprechend dem "Instruction Manual" (Fa. Roche) markiert. Nach dem Waschen wird die DIG-markierte Nucleinsäure mit den Anti-DIG Fab-Fragmenten konjugiert an alkalische Phosphatase; Verdümung 1:10,000] detektiert und durch Chemolumineszenztechnik unter Benutzung von CDP entsprechend den Instrultionen, des Herstellers (Fa. Roche) sichtbar gemacht. Die Screens können danach zur Auswertung z. B. mit einem GS-525 Molecular Imager (Fa. Bio-Rad) gescannt werden.

Die Northern-Blot-Untersuchingen ergaben, daß die Mengen an Transkripten nach Überführung der Tiere von Silicat/Fe(+++)-freiem in Silicat/Fe(+++)-enthaltendes Seewasser stark ansteigt (Abbildung 4). Parallele Hybridisierungsstudien mit dem *SDTUB* (β-Tubulin) Gen zeigten, daß dieselbe an RNA auf die Gele geladen wurde.

Aufgrund dieser Ergebnisse kann geschlossen werden, daß in intakten Tieren nach Inkubation der Tiere mit Silicat/Fe(+++) *silicatein-*β-positive Zellen gebildet werden.

### In situ-Lokalisierungsstudien

Die Expression des *Silicatein*-β-Gens kann auch mittels *in situ*-Hybridisierung verfolgt werden. Hierzu wird Gewebe von Tieren z.B. nach 6-tägiger Inkubation in einem Silicat/Fe(+++)-Medium für 30 min in Seewasser, ergänzt mit 30 mM Ethylendiamintetraessigsäure (EDTA), bei Raumtemperatur behandelt. Die Spiculae werden dann durch Sedimentation erhalten und für die *in situ*-Hybridisierung weiter verarbeitet.

Im folgenden wird eine Methode angewandt, die auf der von Polak & McGee (In situ Hybridization. Oxford University Press, Oxford, 1998) beschriebenen Prozedur mit Modifikationen (Le Pennec et al. (2003) J Biotechnol 100:93-108) basiert. 8-µm dicke gefrorene Schnitte werden bei -30°C mit Hilfe eines Cryostats erhalten. Die Cryoschnitte werden mit Paraformaldehyd fixiert und dann 2-mal mit 1xPBS bei Raumtemperatur gewaschen. Die Schnitte werden mit Proteinase K inkubiert und anschließend wieder mit Paraformaldehyd fixiert. Um die Schwammfarbe zu entfernen, werden die Schnitte mit Ethanol und letzendlich mit Isopropanol inkubiert. Nach der Rehydrierung mit 1xPBS werden die Digoxigenin(DIG)-markierten DNA-Sonden zu der Hybridisierungslösung hinzugefügt. Die Hybridisierung wird über Nacht in einem Glasbehälter bei 45°C durchgeführt; die anschließenden Waschschritte werden bei 50°C wie beschrieben durchgeführt (Perović et al. (2003) Evo & Devo 5:240-250). Nach dem Blocking werden die Schnitte z.B. mit einem Anti-Digoxigenin-Antikörper, der mit alkalischer Phosphatase konjugiert ist, inkubiert. Zur Sichtbarmachung der Signale kann das Farb-reagens NBT/X-Phosphat benutzt werden.

### Herstellungt von DNA-Sonden für in situ-Lokazlisierungsstudien

Die *In-situ*-Hybridisierung kann zum Beispiel mit einer Digoxigenin-markierten ssDNA-Sonde durchgeführt werden. Die Sonde Wird z.B. mit dem "PCR DIG Probe synthesis Kit" (Fa. Roche) markiert. Die DNA-Sonde wird basierend auf der *S*. *domuncula* cDNA-Sequenz konstruiert. Sowohl antisense- als auch sense-Sonden werden durch Polymerasekettenreaktion (PCR) unter Benutzung der linearisierten cDNA hergestellt. Die antisense-Sonde wird durch Anwendung eines "forward primer" in 5' nach 3' sense-Richtung erhalten; die komplementäre sense-Sonde wird durch Benutzung eines reverse Primer in 3' nach 5' Orientierung erhalten. Die *SDSILICA*β-Sonde überspannt ein Segment innerhalb des offenen Leserasters mit einer Länge von 520 bp (nt₆₇₆ bis nt₁₁₉₈). Die PCR wird zum Beispiel mit Hilfe eines GeneAmp 9600 thermal cycler (Perkin Elmer) durchgeführt. Folgende Reaktionsbedingungen haben sich bei der PCR als geeignet erwiesen: initiale Denaturierung bei 95°C für 3 min, dann 35 Amplifikationszyklen jeweils bei 95°C für 30 s, 58°C - 30 s, 74°C - 4 min und ein finaler Extensionsschritt bei 72°C für 20 min. Die Markierung kann zum Beispiel mit Hilfe des "DIG Oligonucleotide Labeling Kit" (Fa. Roche) erfolgten.

### Silicatein-positive Zellen

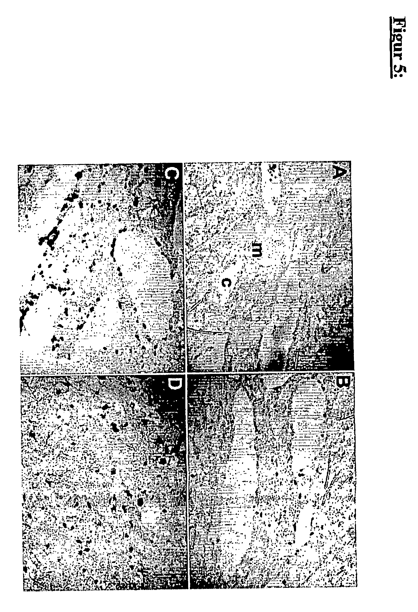

In dem in Abbildung 5 wiedergegebenen Experiment wurde mit Hilfe der *In-situ-*Hybridisierungsstudien gezeigt, daß keine *Silicatein*-positiven Zellen in Gewebe von Schwämmen, die in Silicat/Fe(+++)-freiem Seewasser gehalten wurden, vorhanden sind. Interessanterweise erscheinen die *silicatein*-positiven Zellen zunächst in der Epithelschicht (Pinacoderm), und erst später, vier Tage nach der Silicat/Fe(+++)-Exposition, werden auch Zellen im Mesohyl mit der *silicatein*-Sonde positiv (B - D).

### 3. Herstellung des Silicatein-β-Polypeptids

Das Silicatein-β-Polypeptid kann aus Geweben oder Zellen gereinigt oder rekombinant hergestellt werden.

### 3.1. Reinigung des Silicatein-β-Polypeptids aus natürlichen Quellen

Die Reinigung des Silicatein-β kann vorteilhaft aus isolierten Spiculae von Schwämmen durchgeführt werden. Mit Hilfe dieser Prozedur kann das Silicatein-β-Polypeptid unter anderem aus dem Schwamm *S*. *domuncula* gereinigt werden.

Hierzu werden aus dem Schwamm, z. B. *Suberites domuncula*, die Spiculae (bestehend aus amorphem Silicat) durch Dissoziation des Gewebes in Ca⁺⁺- und Mg⁺⁺-freiem Seewasser und Sedimentation gewonnen. Das amorphe Silicat der Spiculae wird im alkalischen Milieu, z. B. in verdünnter Natronlauge, entfernt. Die organischen Fibrillen der Spiculae, die Silicatein-β enthalten, werden durch Abzentrifugation (z. B. 20,000 x g; 1 Stunde; 4°C) gewonnen. Das Protein wird durch hohe Salzkonzentration, wie z. B. 1 M NaCl, aber auch durch den "Protein Refolding-Kit" in Lösung gebracht.

Anschließend wird das Silicatein-β an einer Affinitätsmatrix gereinigt. Die Affinititätsmatrix wird hergestellt, indem ein Silicatein-β-spezifischer Antikörper an eine feste Phase (CNBraktivierte Sepharose oder andere geeignete Träger) immobilisiert wird, gereinigt. Als Antikörper werden monoklonale oder polyklonale Antikörper gegen das Silicatein-β eingesetzt, die nach Standard-Methoden hergestellt werden (Osterman, L.A. Methods of Protein and Nucleic Acid Research Vol. 2; Springer-Verlag [Berlin] 1984). Die Kopplung des Antikörpers an die Säulenmatrix wird nach den Angaben des Herstellers (Fa. Pharmacia) durchgeführt. Die Elution des reinen Silicatein-β erfolgt mittels pH-Änderung oder Änderung der Ionenstärke.

### 3.2. Herstellung von rekombinantem Silicatein-β-Polypeptid

### 3.2.1. Klonierung der cDNA aus marinen Schwämmen

Die Clonierung der Silicatein-β-cDNA aus dem Meeresschwamm *S*. *domuncula* wurde oben beschieben.

Das Silicatein-β-Gen kann auch aus cDNA-Bibliotheken, z. B. in *ZapExpress* und in *Escherichia coli* XL1-Blue MRF', mit geeigneten degenerierten Primern mittels der PCR-Technik identifiziert werden; hierzu werden entsprechende vektorspezifische Primer eingesetzt. Die erhaltenen Syntheseprodukte werden zum Screenen in den betreffenden cDNA-Bibliotheken benutzt. Danach werden die identifizierten Clone in einen Vektor (beispielsweise *pGem-T*) subcloniert und anschließend sequenziert.

### 3.2.2. Expression und Isolierung des rekombinanten Silicatein-β-Polypeptids

Die Hersbellung von rekombinantern Silicatein-β-Polypeptid erfolgt bevorzugt in *E. coli*. Aber auch die Herstellung in Hefen und Sängerzellen ist möglich und wurde erfolgreich durchgeführt. Hierzu wird die cDNA in einen entsprechenden Vektor, z. B. *pQE-30*, eincloniert. Nach Transformation von *E*. *coli* wird die Expression des Silicatein-β-Polypeptids durch Induktion mit IPTG (Isopropyl-β-D-thiogalactopyranosid) (Ausubel et al. (1995) Current Protocols in Molecular Biology. John Wiley and Sons, New York) durchgeführt. Die Expression des Silicatein-β-Polypeptids sowie die Aufreinigung des rekombinanten Proteins über z. B. das Histidin-Tag, das an dem rekombinanten Protein vorliegt, kann an entsprechenden Affinitätssäulen, z. B. einer Ni-NTA-Matrix durchgeführt werden (Skorokhod et al. (1997) Cell. Mol. Biol. 43:509-519).

Im Folgenden ist als Beispiel die Expression des Silicatein-β-Gens von *S*. *domuncula* in *E*. *coli* unter Benutzung des "GST (Glutathion-S-Transferase) Fusions"-Systems (Fa. Amersham) beschrieben. In dem Beispiel wird ein Insert benutzt, das lediglich die Aminosäuren aa₄₈ bis aa₃₈₃ (kurze Form) umfaßt; ebenso kann auch ein Insert benutzt werden, welches das gesamte abgeleitete Protein umfaßt. Der entsprechende Klon wird in einen Vektor eincloniert, z. B. in das Plasmid *pGEX-4T-2,* welches das GST-Gen von *Schistosoma japonicum* enthält. Auch andere Expressionsvektoren haben sich als geeignet erwiesen. Nach Transformation von *E*. *coli* wird die Expression des Silicatein-β-Polypeptids üblicherweise durch IPTG induziert und für 4 oder 6 Stunden bei 37°C durchgeführt (Ausubel FM, Brent R, Kingston RE, Moore DD, Smith JA, Seidmann JG, Struhl K (1995) Current Protocols in Molecular Biology. John Wiley and Sons, New York). Das erhaltenen GST-Fusionsprotein wird z. B. durch Affinitätschromatographie an Glutathion-Sepharose 4B gereinigt. Zur Abtrennung der Glutathion-S-transferase von dem rekombinanten Schwamm-Silicatein-β-Polypeptid wird das Fusionsprotein mit Thrombin (10 Units/mg) gespalten. Das Protein wird dann der Gelelektrophorese in Gegenwart von 2-Mercaptoethanol unterworfen. Die Gelelektrophorese kann in 10%igen Polyacrylamidgelen mit 0,1% NaDodSO₄ (Polyacrylamidgel-Elektrophorese; PAGE) durchgeführt werden. Das Gel wird mit Coomassie Brillant Blau gefärbt. Nach der Spaltung, Reinigung und anschließender PAGE wird die kurze Form des rekombinanten Proteins erhalten.

### 3.2.3 Expression und Isolierung des rekombinanten Silicatein-β-Polypeptids aus weiteren Organismen

Entsprechend der oben beschriebenen Vorgehensweise kann die Isolierung, Clonierung und Expression der Silicatein-β-cDNA auch aus anderen Organismen durchgeführt werden, beispielsweise aus (Siliciumdioxid-produzierenden) Hexactinelliden (z. B. *Rhabdocalyptus dawsoni).*

### 3.3. Isolierung und Reinigung des Silicatein-β-Polypeptids mittels Antikörper

Nach Extraktion oder partieller Renigung nach einem der oben geschilderten Verfahren wird das Silicatein-β an einer Antikörper-Affinitätsmatrix gereinigt. Die Vorgehensweise entspricht derjenigen, die in Abschnitt 3.1 ("Reinigung des Silicatein-β-Polypeptids aus natürlichen Quellen") beschrieben wurde.

Auch andere Affinitätsmatrizes wie polymere Silicate oder copolymere Silicate/Germanate wurden mit Erfolg eingesetzt.

### 4. Nachweis der Silicatein-β-Aktivität und Synthese von Silicium-Alkoxy-Verbindungen

Im Folgenden werden lediglich die Aktivitäten, die für die kurze Form der rekombinanten Schwamm-Silicatein-β-Polypeptids gefunden werden, angegeben.

### 4.1. Silicatein-β-Aktivität

### 4.1.1. Polymer (z.B. Silica)-bildende Aktivität

Zur Bestimmung der Enzymaktivität des rekombinaten Silicatein-β kann ein Assay angewandt werden, der auf der Messung von polymerisiertem und präzipitiertem Silicat nach Hydrolyse und nachfolgender Polymerisation von Tetraethoxysilan (TEOS) basiert.

Die Messung der enzymatischen Synthese-Aktivität des rekombinanten Silicatein-β wird üblicherweise wie folgt durchgeführt. Das rekombinante Silicatein-β wird über Nacht gegen einen für die Reaktion geeigneten Puffer, wie 50 mM MOPS, pH 6,8 dialysiert [andere Puffer innerhalb eines pH-Bereiches von 4,5 bis 10,5 sind ebenfalls geeignet].

I - 50 µg rekombinantes Silicatein-β werden in 1 ml eines geeigneten Puffers, wie 50 mM MOPS (pH 6,8) gelöst und mit 1 ml einer 1 - 4,5 mM Tetraethoxysilan-Lösung versetzt. Die enzymatische Reaktion kann bei Raumtemperatur durchgeführt werden. Bei einer Inkubationszeit von 60 min werden üblicherweise 200 nmol amorphes Silicat (als Molybdat-reaktives, lösliches Silicat) pro 100 µg Silicatein-β synthetisiert. Zum Nachweis der Silicat-Produkte wird das Material in einer Tischzentrifuge abzentrifugiert (12 000 x g; 15 min; +4°C), mit Ethanol gewaschen und luftgetrocknet. Anschließend wird das Sediment mit 1 M NaOH hydrolysiert. In der entstandenen Lösung wird unter Anwendung eines Molybdat-gestützten Nachweisverfahrens, wie z. B. dem Silicon-Assays (Merck), Silicat quantitativ gemessen.

Überraschenderweise wurde gefunden, daß Silicatein-β neben dem Substrat Tetraethoxysilan auch noch weitere Silan-Alkoxide polymerisiert.

Folgende Verbindungen können als Reaktanten (Substrate) zur Carboxypeptidasevermittelten Synthese verwendet werden: Tetraalkoxysilane, Trialkoxysilanole, Dialkoxysilandiole, Monoalkoxysilantriole, Dialkoxysilanole, Monoalkoxysilandiole, Monoalkoxysilanole, Alkyl-, Aryl- oder Metallo-Trialkoxysilane, Alkyl-, Aryl- oder Metallo-Silanole, Alkyl-, Aryl- oder Metallo-Silandiole, Alkyl-, Aryl- oder Metallo-Silantriole, Alkyl-, Aryl- oder Metallo-Monoalkoxysilandiole, Alkyl-, Aryl- oder Metallo-Dialkoxysilanole, oder andere Metall(IV)-Verbindungen (Alkoxyverbindungen von Gallium (IV), Zinn (IV) oder Blei (IV). Auch Mischungen dieser Substrate werden durch das Enzym erkannt und polymerisiert. Somit können auch Mischpolymere hergestellt werden.

Die Substrate, wie Tetraethoxysilan, werden in Dimethylsulfoxid in einer Stammlösung von üblicherweise 500 mM gelöst und anschließend in die gewünschte Endkonzentration herunter verdünnt.

Die Silicatein-Reaktion läßt sich auch mit anderen, aus der Silicium-Chemie her bekannten Reaktionen kombinieren, wie der Müller-Rochow-Synthese von Chlormethylsilanen, der Synthese längerkettiger Silane (z.B. Si₈-Silane) und Silicium-Stickstoff-Verbindungen (z.B. Siliciumnitrit, Si₃N₄). Die letztgenannte Verbindung entsteht bei der Verbrennung von Silanen durch Stickstoff oder Luft (Luft-Stickstoff). Längerkettige Silane entstehen pyrolytisch, z.B. in Gegenwart von Kupfer bei 50°C. Über das Müller-Rochow-Verfahren (Katalysator: Kupfer; Temperaturen zwischen 250 -300°C) erfolgt auch überwiegend die großtechnische Synthese von Methylsiliconen. Diese Verfahren sind Stand der Technik. Niedere Silane sind sehr unbeständig und entzünden sich leicht -unter heftigen Explosionen-an Luft oder bei Kontakt mit Wasser, während höhere (längerkettige) Silane, die nicht mehr selbstentzündlich sind (ab n-Heptasilan), vielverspechende Energieträger (Brennstoffe oder Treibstoffe) darstellen. Verbrennungsprodukt dieser Silane ist - ungiftiges - Siliziumnitrit (Si₃N₄). Da die durch Silicatein katalysierte enzymatische Reaktion Vorstufe zu diesen Silanen liefert, ergibt sich eine deutliche Erleichterung und Erhöhung der Effizienz und Spezifität von deren Synthese.

Als ein Beispiel, das den Umfang der Patentansprüche nicht einschränkt, wird folgendes Reaktionsschema gegeben: Dabei werden kurzkettige Silicate mit einer Kettenlänge von Siₙ mit n > 8 durch den Biokatalysator Silicatein hergestellt. Die Hydroxylgruppen in diese kurzkettigen Silicaten werden in einer elektrisch induzierten Hydrolyse mit Wasserstoff, einem Reduktionmittel, welches Wasserstoff freisetzt, oder durch Lewis-Säuren katalysierte Hydrierungen ersetzt. Das frei werdende Wasser wird aus dem Reaktionsgemisch durch geeignete Verfahren, entsprechend dem Stand der Technik für Reduktions- und Trocknungsverfahren, oder durch Destillationsverfahren entfernt. Die Reaktionskette kann formal wie folgt dargestellt werden:
(1) n>8 Si(OH)₄ + Silicatein → Si_{n>8}-(OH)_{2n>8}-(OH)₂ + n>8 H₂O
   (bei Raumtemperatur - neutral pH)
(2) Si_{n>8}-(OH)_{2n>8}-(OH)₂ + 2n>8+2 H_{2,aktiviert (nascendi)} → Si_{n>8}H_{2n>8+2}
   (unter Wasserentzug und Elektroaktivierung)

Erfmdungsgemäß kann ein Zwei-Stufen-Prozeß aufgebaut werden. Elektrochemisch kann Wasserstoff aus Wasser hergestellt werden. Dazu sind eine Vielzahl von Verfahren bekannt. Parallel dazu, in einer Apparatur aber räumlich getrennt, werden erfindungsgemäß durch die katalytischen Polypeptide Silicatein α und β Oligosilicate mit Siₙ(OH)₂ₙ₊₂ mit n ≥ 7 unter - im Gegensatz zum Stand der Technik - extrem milden Bedingungen aufgebaut. Nach Entfernung der Reaktionslösung können in einer anschließenden Reaktion mit aktivierten Wasserstoff_{nas-cendi} die Oligosilicate zu Oligosilanen entsprechend der Formel Siₐ(H)₂ₙ₊₂ mit n≥7 durch Hydrierung überführt werden. Diese Reaktionsführung ist möglich, da Silane ab n=7 entsprechend der Formel Siₙ(OH)₂ₙ₊₂ nicht mehr spontan mit Lüftsauerstoff oder Wasser reagieren, da ihr Aktivierungsenergiepotential mit zunehmender Zahl n steigt. Bei den niederen Silanen findet dagegen aufgrund des niedrigen Aktivienmesenergiepotentials eine spontane Reaktion statt.

Die vorliegende Erfindung trägt damit auch zur Einführung energiesparender und umweltfreundlicher Verfahren bei, da unter anderem auf die derzeit verwendeten chlorierten Silicatverbindungen verzichtet werden kann. Ferner kann die gesamte Reaktionsführung unter extrem milden Reaktionsbedingungen durchgeführt werden. Durch die vorliegende Erfindung können Oligosilicate als Wasserstoffspeicher genutzt werden, der universell, unter geringen Gefahren und mit einfachem technischen Aufwand transportierbar und lagerbar ist.

Erfindungsgemäß können mit Silicatein α und β, als katalytische Polypetide, wie zuvor dagestellt, kurzkettige Oligosilicate mit n > 2 hergestellt werden. Im Gegensatz zum Stand der Technik, bei dem Temperaturen von >200°C und Drücke über Normaldruck verwendet werden, reichen hier Temperaturen unter 50°C und Normaldruck aus. Ferner kann die Reaktion im wäßrigen Medium durchgeführt werden, was nach dem Stand der Technik nicht möglich ist. Dies bedeutet eine erhebliche Verbesserung der Umweltverträglichkeit, der Sicherheit und auch eine Einsparung von Kosten. Die Oligosilicate sind in nachfolgenden Reaktionsführungen Grundlage für eine Vielzahl von derzeit verwendeten Produkten, wie Silanen, Siliconen und anderen organischen Siliciumverbindungen.

Die Silicatein-Reaktion erlaubt aufgrund ihrer Reversibilität auch eine Mischether-Herstellung (Bildung von Si-O-C Bindungen), wie oben dargelegt. Aus diesen Verbindungen lassen sich - nach dem Stand der Technik - mit Hilfe anderer Katalysatoren Halogenverbindungen (z.B. mit Chlor) bilden, aus denen unter Wasserstoff-Atmosphäre unterschiedliche Silanverbindungen gewonnen werden können.

Silicatein ist insbesondere auch in der Lage, Germanium und Titan-Verbindungen zu synthetisieren und abzubauen, und eignet sich auch zur Herstellung von Si-Ti, Si-Ge oder Si-Ge-Ti Mischstrukturen, die für die Chip-Produktion von Bedeutung sind.

### 4.1.2. Polymer (z.B. Silica)-abbauende Aktivität (Silica-auflösende Aktivität)

Silicatein-β ist auch in der Lage, Silica aufzulösen, insbesondere wenn die Reaktion in Anwesenheit von Ascorbinsäure oder einer anderen, Catechol oder 1,2-Diphenol-enthaltenden Verbindung stattfindet. Als Substrat für Silicatein-β können beispielsweise Spiculae (amorphes *Siliciumdioxid) von S. domuncula dienen.*

Die Spiculae können aus Schwammgewebe durch 12-stündige Inkubation in Gegenwart von Ethylendiamintetraessigsäure (20 mM, in PBS; PBS = Phosphatpuffer-Salz-Lösung, bestehend aus 1,15 mM KH₂PO₄, 8,1 mM Na₂HPO₄, 137 mM NaCl und 2,7 mM KCl) erhalten werden. Nach Waschen mit destilliertem Wasser und mit Ethanol (zweimal) werden die Spiculae getrocknet (56°C) und dann in einem Mörser zu einem Pulver zerrieben.

Die Silica-auflösende Aktivität kann dann wie folgt bestimmt werden. Üblicherweise werden 100 µg der getrockneten Spiculae (Pulver) zu einem geeigneten Puffer wie 50 mM Tris-HCl-Puffer (pH 7,2; 10 mM DL-Dithiothreitol, 100 mM NaCl) und 0,5 mM ZnSO₄ in 2 ml Eppendorf-Gefäßen hinzugegeben. Dann werden üblicherweise 50 µl des rekombinanten Silicatein-β-Polypeptids hinzugefügt und bei 25°C inkubiert (die Inkubation ist auch bei anderen Temperaturen zwischen 5°C und etwa 65°C möglich). Die durchschnittliche Inkubationszeit beträgt 60 Minuten. Zur quantitativen Bestimmung der Menge an gelöstem Siliciumdioxid werden die nichtgelösten Spiculae abzentrifugiert (14000 x g; 15 Minuten; 4°C). Die freigesetzte, lösliche Kieselsäure kann z. B. mit Hilfe eines Molybdat-gestützten Nachweisverfahrens, wie z. B. dem kolorimetrischen "Silicon Test" (Merck; 1.14794), quantitativ bestimmt werden. Die Menge an Kieselsäure wird in diesem Fall anhand einer Kalibrierungskurve mit einem Siliciumstandard (Merck 1.09947) aus den Extinktionswerten bei 810 nm berechnet.

### 4.1.3. Silicium (IV)-Verbindung-synthetiserende Aktivität

Die Reversibiltät der Alkoxysilan-hydrolysierenden Aktivität des Silicatein-β (aber auch weiterer Silicateine wie Silicatein-α) kann auch zur Synthese anderer Silicium (IV)-Verbindungen oder anderer Metall(IV)-Verbindungen benutzt werden, und zwar dadurch, daß die einzuführende, vorteilhaft nucleophile Gruppe der das Alkoxysilan (Alkoxy-Metall(IV)-Verbindung) und das Enzym enthaltenden Reaktionsmischung hinzugesetzt wird. Neben Tetraalkoxysilanen wie z.B. Tetraethoxysilan (TEOS) können auch Trialkoxysilane, Dialkoxysilane und Monoalkoxysilane sowie Trialkoxysilanole, Dialkoxysilandiole, Dialkoxysilanole, Monoalkoxysilantriole, Monoalkoxysilandiole, Monoalkoxysilanole sowie Alkyl-, Aryl- oder Halogen-substituierte Alkoxyverbindungen des Silicium (IV) eingesetzt werden.

Die Silicium (IV)- Verbindung-synthetiserende Aktivität des Silicatein-β kann auch mit der Polymerisation dieser Substrate oder Mischungen dieser Substrate oder der aus ihnen entstehenden Produkte verknüpft werden, wobei eine Durchführung der Reaktionen simultan im gleichen Ansatz möglich ist. Somit können auch unterschiedliche Polymere oder Mischpolymere hergestellt werden.

### 4.1.4. Gekoppelter optischer Test zur Bestimmung der Silicatein-Reaktion

Ein einfacher Test zur Bestimmung der enzymatischen Aktivität des Silicateins wird angegeben. Hierbei handelt es sich um einen gekoppelten optischen Test zur Bestimmung des bei der durch Silicatein vermittelten Enzymreaktion aus Substraten (Alkoxy-Silanen und Derivaten) freigesetzten Alkohols. Benötigt wird hierzu ein Photometer (z.B. Eppendorff Photometer 1101 M). Als Pufferlösungen werden verwendet:

### Lösung 1

2 mmol/l ABTS (Azino-bis (3-Ethylbenzthiazoline-6-sulfonic acid)) in Puffer, hergestellt wie folgt:
Schritt a: 0,1 M Kaliumphosphat Puffer (pH 7,5), hergestellt aus Lösung A (8,16 g KH₂PO₄ in Aqua Bidest lösen, auf 600 ml auffüllen) und Lösung B (68,4 g K₂HPO₄ in Aqua Bidest lösen, auf 300 ml auffüllen; =10x Ansatz), und zwar werden 300 ml Lösung A mit Lösung B auf pH 7,5 gebracht.
Schritt b: 320 ml der in Schritt a hergestellten Lösung werden 30 min mit O₂ gesättigt; dann werden darin 352 mg ABTS gelöst. (Bemerkung: ABTS ist licht- und luftempfindlich, Puffer nicht länge als 1 h benutzen).

### Lösung 2

POD (Peroxidase, Fa. Roche); 10 mg Protein/ml, spezifische Aktivität ca. 5 U/mg.

### Lösung 3

Alkoholoxidase (Fa. Sigma)-Lösung, hergestellt wie folgt:
Schritt a: 100 mg BSA werden in 0,1 M Kaliumphosphat Puffer (pH 7,5) gelöst.
Schritt b: 10 mg Alkoholoxidase-Lyophilisat werden in 1 ml des in Schritt a hergestellten BSA-0,1 M Kaliumphosphat-Puffers (kalt) gelöst.
Schritt c: 135 µl der in Schritt b hergestellten Alkoholoxidase-Lösung werden mit 10 ml des in Schritt a hergestellten BSA-0,1 M Kaliumphosphat-Puffers (kalt) verdünnt.

### H₂O₂

5 µl (Fa. Merck) werden mit Aqua Bidest auf 50 ml verdünnt.

Als Substratlösung kann beispielweise 4,5 mM Tetraethoxysilan (TEOS) in MOPS-Puffer (100 mM NaCl, 5 mM CaCl₂, 0,1 mM ZnSO₄, pH 6,8) benutzt werden.

Die Testdurchführung ist wie folgt:
In Küvette pipettieren:
   2,8 ml Puffer/ABTS
   10 µl POD
   50 µl AO
Dann mischen, und danach Zugabe von
10 µl H₂O₂
Erneut mischen, Nullabgleich des Photometers (auf 0,1 einstellen), dann
Zugabe von 100 µl Substratlösung bzw. Enzym (Silicatein) in Substratlösung, mischen, Extinktion über mindestens 2 min verfolgen (Filter 405 nm).

Als Positiv-Kontrolle und für die Eichgerade dienen verschiedene Alkohol (z.B. Ethanol)-Konzentrationen.

### 5. Ligation der cDNA für Silicatein-β mit einer oder mehreren cDNA(s) für anderer Proteine

### 5.1. Herstellung von Silicatein-β-Fusionsproteinen

Zur Herstellung von Fusionsproteinen mit dem Silicatein-β-Polypeptid wird ein geeigneter Expressionsvektor (beispielsweise *pQE*-30-Vektor; Qiagen) eingesetzt. Die Silicatein-β-cDNA - mit z. B. einer *Bam*HI-Restriktionsstelle am 5'-Terminus und z. B. einer *Sal*I-Restriktionsstelle am 3'-Terminus - wird hergestellt. Das Stopp-Codon in der Silicatein-β-cDNA wird entfernt. Hierzu wird die PCR-Technik eingesetzt und zum Amplifizieren Primer, welche die betreffenden Restriktionsstellen besitzen, benutzt. Die cDNA für das zweite Protein wird entsprechend gewonnen, wobei am 5'-Terminus die gleiche Schrnittstelle wie am 3'-Terminus der Silicatein-β-cDNA (im Beispiel *SalI*) und am 3'-Terminus eine von den anderen verschiedene (z. B. eine *Hin*dIII-Stelle) vorliegt Falls sich in den betreffenden cDNAs interne Restriktionsstellen befinden, können alternative Restriktionsenzyme eingesetzt werden. Darüber hinaus können auch Linker zwischen die beiden cDNAs eingesetzt werden.

Die beiden cDNAs werden nach den üblichen Verfahren ligiert, gereinigt und in *den pQE-30-*Vektor einligiert. Die Ligation erfolgt im Anschluß an das Histidin-Tag (etwa 6 Histidin-Codons). Die Expression und Reinigung des Fusionsproteins über z. B. das Histidin-Tag, das an dem rekombinanten Protein vorliegt, kann an entsprechenden Affinitätssäulen, z. B. einer Ni-NTA-Matrix, durchgeführt werden (Skorokhod A, Schäcke H, Diehl-Seifert B, Steffen R, Hofmeister A, Müller WEG (1997) Cell Mol Biol 43:509-519).

### 5.2. Getrennte Expression I (Protease-Spaltstelle)

Alternativ zu dem Verfahren unter 5.1. kann zwischen der cDNA für das Silicatein-β-Polypeptid und der cDNA für ein weiteres Protein eine Protease-Spaltstelle (wie z. B. eine Enterokinase-Stelle) einkloniert werden. In diesem Falle kann ein Codon für ein neues Start-Methionin vor den codierenden Bereich des Gens für das weitere Protein insertiert werden. Nach Expression und Reinigung wird das Fusionsprotein proteolytisch gespalten. Jetzt liegen beide Proteine separat vor.

### 5.3. Getrennte Expression II (Kassetten-Expression)

Alternativ können beide Proteine auf einem Konstrukt separat exprimiert werden. Hierzu wird in einem Expressions-Vektor das Silicatein-β-Gen dem His-Tag nachgeschaltet. Am Ende der Silicatein-β-cDNA wir ein Stopp-Codon insertiert. Zwischen der cDNA für das Silicatein-β-Polypeptid und der cDNA für das weitere Protein wird eine Ribosomen-Bindungsstelle mit Codon für ein Start-Methionin eincloniert. Wiederum wird ein His-Tag der cDNA für das weitere Protein vorgeschaltet. Ebenfalls erhält dieses Gen ein Stopp-Codon.

Die His-Tags können deletiert werden, wenn die Proteine zur Funktionsanalyse in den betreffenden Wirtszellen benutzt werden.

### 5.4. Erweiterungen

Für die unter 5.1 bis 5.3 beschriebene Expression können sowohl bakterielle als auch eukaryotische Zellen benutzt werden.

Die unter 5.1 bis 5.3 beschriebene Expression kann auch für drei und mehr offene Leserahmen eingesetzt werden.

### 6. Verwendungen der Silicatein-β-Polypeptids und der Silicatein-β-Fusionsproteine

Ein weiterer Aspekt der Erfindung sind die nachstehend genannten Verwendungen des rekombinanten Silicatein-β-Polypeptids, des aus verschiedenen Quellen gereinigten Silicatein-β und der Silicatein-β-Fusionsproteine. Die diesen Verwendungen zugrundeliegenden Verfahren sind für den Fachmann aus der hier und oben bereits angegebenen Beschreibung, der Fachliteratur und mittels des allgemeinen Fachwissens ohne weiteres herzuleiten.
1.) Verwendung zur Oberflächenmodifikation von Biomaterialien (Verbesserung der Biokompatibilität). Oberflächen-modifizierte Biomaterialien finden u. a. Verwendung zur Beeinflussung von Zelladhäsion und Wachstum, zur Modifizierung der Blut-Kompatibilität oder zur Kontrolle der Protein-Adsorption (z. B. Herabsetzung der Adsorption von Kontaktlinsen). Eine Literatur-Übersicht findet sich in: Ratner BD et al (Hrsg) Biomaterials Science - An Introduction to Materials in Medicine. Academic Press, San Diego, 1996. Ein Problem besteht darin, daß die zur Herstellung dieser Modifikationen angewandten Bedingungen oft einen schädlichen (destruierenden) Effekt auf die benutzten Biomaterialien haben. Eine im Vergleich zu den benutzten physikalisch/chemischen Verfahren "milde", Biomaterialien schonenden Methode stellt eine Modifikation der Oberflächen dar, die allein auf biochemisch/enzymatischen Reaktionen beruht, was mit Hilfe des erfindungsgemäßen Verfahrens ermöglicht wird (Silicatein-β-vermittelte enzymatische Synthese und - als reversible Reaktion - enzymatischer Abbau von SiO₂- oder Siloxan-enhaltenden Oberflächen mit Hilfe des rekombinanten/gereinigten Silicatein-β-Polypeptids). Insbesondere ergibt sich auch eine Verwendung des rekombinanten oder aus natürlichen Quellen gereinigten Silicatein-β bei der Herstellung von Oberflächen-Modifikationen (beim Coating) von Silicon-Materialien, wie Silicon-Brust-Implantaten, Endoprothesen oder Metall-Implantaten (Verbesserung der Verbindung zwischen Knochen und Metall-Implantat, Biologisierung der Metall-Implantate) sowie Kontakt/Plastiklinsen. Weitere Verwendungen betreffen das Coating von Collagen, das als Knochenersatzmaterial dient, und von Collagen-Vliesen, die z. B. für das "Tissue Engineering" benutzt werden. Das Ziel ist hierbei die Erhöhung der Stabilität und der Porosität sowie die Verbesserung der Resorbierbarkeit.
2.) Verwendung zur Herstellung neuer Biomaterialien wie Knochenersatzmaterialien oder Zahnersatzmaterialien durch Co-Synthese von Polysilicaten, Siliconen oder Mischpolymeren.
3.) Verwendung zur Oberflächen-Modifikation (Kontaktzonen-Behandlung) von (Silicium)-Halbleitern oder Silicium-Chips.
4.) Verwendung zur Modifikation oder zur Synthese von Nano-Strukturen aus amorphem Siliciumdioxid. Mittels des rekombinanten Silicatein-β, der rekombinanten Silicatein-β-Fusionsproteine oder des gereinigten Silicatein-β ist es möglich, spezifische zwei- und dreidimensionale Strukturen aus amorphem Siliciumdioxid im Nano-Maßstab zu modifizieren oder zu synthetisieren. Die gebildeten Strukturen können in der Nanotechnologie angewandt werden.
5.) Verwendung zur Oberflächenmodifikation von Silicium-haltigen Edelsteinen und Halbedelsteinen. Zu den amorphen bzw. feinkristallinen Modifikationen des SiO₂ zählen u. a. Achat, Jaspis und Onyx. Aufgrund der Möglichkeit, mit Hilfe des Silicatein-β unter kontrollierten Bedingungen die Oberfläche dieser Minerale zu modifizieren, ergibt sich die Verwendung des erfindungsgemäßen Verfahrens bei der Herstellung oder Bearbeitung dieser Edelsteine/Halbedelsteine. Hierbei ergibt sich auch die Möglichkeit, gezielt Fremdmoleküle/atome einzufügen.
6.) Verwendung zur Herstellung von Überzügen für Metalle, Metalloxide, Kunststoffe und andere Materialien; insbesondere zur Herstellung von monomolekularen Schichten auf diesen Materialien.
7.) Verwendung zur Herstellung von Überzügen für technische Fasermaterialien, z. B. Carbonfasern, zum Feuerschutz, zum Zwecke einer besseren Verarbeitung oder weiteren Veränderungen der Eigenschaften dieser Materialien.
   Carbonfasern (Kohlenstoffasern) besitzen eine große technische Bedeutung; da durch ihren Einsatz im Vergleich zu Metallen wesentlich leichtere, aber trotzdem festere und steifere Bauteile hestellbar sind (wichtig insbesondere in der Luft-und Raumfahrt).
8.) Verwendung zur Herstellung von Überzügen für Wolle oder Baumwolle zur Erzielung neuer Eigenschaften, insbesondere antiallergischer Eigenschaften, einer Verbesserung der Reinigung oder weiteren Veränderungen der Eigenschaften dieser Materialien. Gerade hier zeigt sich der Vorteil eines enzymatischen Verfahrens, da diese Materialien empfindlich gegenüber den bei den heutigen Verfahren notwendigen drastischen Bedingungen sind. Mit Hilfe der Silica-Beschichtung kann verhindert werden, daß bei der Reinigung Bestandteile der hierbei verwendeten Lösungen an den Materialien hängen bleiben.
9.) Verwendung zur Herstellung von Überzügen/Beschichtungen zur Herabsetzung allergischer Reaktionen bei Zusatzstoffen (z.B. Stärke) zu Medikamenten (Tabletten).
10.) Verwendung zur Herstellung von Silicium-Stickstoff-Verbindungen.
11.) Verwendung zur Synthese von Silicium-organischen Verbindungen.
12.) Verwendung zur Herstellung von Silica-Komplexen mit Polyphosphaten, die auch über divalente Kationen verbunden sein können.
13.) Verwendung zur Herstellung von thixotropen Materialien. Silicatein läßt sich auch zur (enzymatischen oder teilenzymatischen) Herstellung thixotroper Materialien verwenden. Diese Materialien besitzen die Eigenschaft, daß ihre Viskosität beim Schütteln oder Rühren - mit einer gewissen zeitlichen Verzögerung - abnimmt (oder auch zunimmt). Hierbei spielt die Bildung und das Aufbrechen von Wasserstoffbrückenbindungen zwischen hydrophilen OH-Gruppen an den Oberflächen von Silikat (Kieselsäure)-Nanopartikeln eine Rolle. Durch geeignete Wahl der Reaktionsbedingungen lassen sich mit Hilfe von Silicatein hochdisperse Kieselsäuren (Silikate) herstellen. Es ist Stand der Technik, daß hochdisperse Kieselsäuren ein effizientes Thixotropierungsmittel darstellen. Thixotrope Flüssigkeiten und Materialien sind von großer technischer Bedeutung (zB. für Anstreicher-Farben).
14.) Erleichterung der Membrangängigkeit vom Medikamenten oder RNAs und DNAs durch Silicatein-vermittelte Überschichtung oder Einkapselung in Silica.

## Patentansprüche

1. Verfahren zur *in vitro synthese* oder *in vivo* Synthese in einer isolierten transfizierten wirtszelle von Siliciumdioxid, Siliconen und anderen Silicium(IV)- oder Metall(IV)-Verbindungen sowie von Mischpolymeren dieser Verbindungen, wobei ein Polypeptid gemäß SEQ ID Nr. 1 oder ein Metallkomplex dieses Polypeptids zur Synthese eingesetzt wird.

2. Verfahren nach Anspruch 1, **dadurch gekennzeichnet, daß** zur Synthese Verbindungen wie Kieselsäuren, Monoalkoxysilantriole, Monoalkoxysilandiole, Monoalkoxysilanole, Dialkoxysilandiole, Dialkoxysilanole, Trialkoxysilanole, Tetraalkoxysilane, Alkyl-, Aryl- oder Metallo-Silantriole, Alkyl-, Aryl- oder Metallo-Silandiole, Alkyl-, Aryl- oder Metallo-Silanole, Alkyl-, Aryl- oder Metallo-Monoalkoxysilandiole, Alkyl-, Aryl- oder Metallo-Monoalkoxysilanole, Alkyl-, Aryl- oder Metallo-Dialkoxysilanole, Alkyl-, Aryl- oder Metallo-Trialkoxysilane oder andere Metall(IV)-Verbindungen als Reaktanten (Substrate) eingesetzt werden.

3. Verfahren zum Abbau von amorphem Siliciumdioxid, Siliconen und anderen Silicium(IV)- oder Metall(IV)-Verbindungen sowie von Mischpolymeren dieser Verbindungen, wobei ein Polypeptid gemäß SEQ ID Nr. 1 oder ein Metallkomplex dieses Polypeptids zum Abbau eingesetzt wird.

4. Verfahren zur Modifikation einer Kieselsäure oder Silicium(IV)- oder Metall(IV)-Verbindung enthaltenden Struktur oder Oberfläche, wobei ein Polypeptid gemäß SEQ ID Nr. 1 oder ein Metallkomplex dieses Polypeptids zur Modifikation eingesetzt wird.

5. Polypeptid eines Silicatein-β aus *Suberites domuncula* gemäß SEQ ID Nr. 1, oder ein Metall-komplex des Polypeptids .

6. Nukleinsäure, **dadurch gekennzeichnet, daß** sie für ein Polypeptid gemäß Anspruch 5 kodiert.

7. Nukleinsäure nach Anspruch 6 in Form eines (a) Fusionsprotein- (chimäres Protein) Konstrukts, (b) Konstrukts mit getrennter Protein-Expression (Protease-Spaltstelle) oder (c) Konstrukts mit getrennter Protein-Expression (Kassetten-Expression).

8. Verfahren zur Auffindung von Inhibitoren oder Aktivatoren eines Polypeptids des Silicatein-β aus *Suberites domuncula* gemäß SEQ ID Nr. 1, wobei a) ein Polypeptid des Silicatein-β aus *Suberites domuncula* gemäß SEQ ID Nr. 1 zur Verfügung gestellt wird, b) das Polypeptid aus Schritt a) mit einem potentiellen Inhibitor oder Aktivator in Kontakt gebracht wird, und c) die Fähigkeit des Polypeptids gemessen wird, Silicate oder Silicone zu synthetisieren oder abzubauen.

9. Verwendung eines Polypeptids oder einer Nukleinsäure nach einem der voranstehenden Ansprüche zur Resorption oder zur Modulation der Resorbierbarkeit von Siliconen und Silicon-Implantaten.

10. Verfahren nach Anspruch 1, **dadurch gekennzeichnet, daß** unter der Verwendung von Silicatein-β und -α eine Synthese von Oligosilicaten mit Siₙ(OH)₂ₙ₊₂ mit n ≥ 2 in wässriger Lösung und/oder wässrig/organischer Lösung, bei Temperaturen unter 50°C und Normaldruck durchgeführt wird.

## Claims

1. A method for the *in vitro* synthesis or the *in vivo* synthesis in an isolated transfected host cell of silicium dioxide, silicones, and other silicium(IV)- or metal(IV)-compounds as well as of mixed polymers of these compounds, wherein a polypeptide according to SEQ ID No. 1 or a metal complex of this polypeptide is used for the synthesis.

2. The method according to claim 1, **characterized in that** compounds such as, for example, silicic acids, monoalkoxy silantrioles, monoalkoxy silandioles, monoalkoxy silanoles, dialkoxy silandioles, dialkoxy silanoles, trialkoxy silanoles, tetraalkoxy silanes, alkyl-, aryl- or metallo-silantrioles, alkyl-, aryl- or metallo-silandioles, alkyl-, aryl- or metallo-silanoles, alkyl-, aryl- or metallo-monoalkoxy silandioles, alkyl-, aryl- or metallo-monoalkoxy silanoles, alkyl-, aryl- or metallo-dialkoxy silanoles, alkyl-, aryl- or metallo-trialkoxy silanes or other metal(IV)-compounds are used as reactants (substrates) for the synthesis.

3. A method for the degradation of amorphous silicium dioxide, silicones and other silicium(IV)- or metal(IV)-compounds as well as of mixed polymers of these compounds, wherein a polypeptide according to SEQ ID No. 1 or a metal complex of this polypeptide is used for the degradation.

4. A method for the modification of a structure or surface containing a silicic acid or silicium(IV)- or metal(IV)-compounds, wherein a polypeptide according to SEQ ID No. 1 or a metal complex of this polypeptide is used for the modification.

5. Polypeptide of a silicatein-β from *Suberites domuncula* according to SEQ ID No. 1 or a metal complex of said polypeptide.

6. Nucleic acid, **characterized in that** it encodes for a polypeptide according to claim 5.

7. Nucleic acid according to claim 6 in form of (a) a fusion protein- (chimeric protein) construct, (b) a construct with separate protein-expression (protease-cleavage site), or (c) a construct with separate protein-expression (cassette-expression).

8. A method for the identification of inhibitors or activators of a polypeptide of the silicatein-β from *Suberites domuncula* according to SEQ ID No. 1, wherein a) a polypeptide of the silicatein-β from *Suberites domuncula* according to SEQ ID No. 1 is provided, b) the polypeptide from step a) is contacted with a potential inhibitor or activator, and c) the ability of the polypeptide is measured to synthesize or to degrade silicates or silicones.

9. Use of a polypeptide or of a nucleic acid according to any of the preceding claims for a resorption or for the modulation of the resorbability of silicones and silicone-implants.

10. The method according to claim 1, **characterized in that** by using silicatein-β and -α a synthesis of oligosilicates with Siₙ(OH)₂ₙ₊₂ with n ≥ 2 in aqueous solution and/or aqueous/organic solution, at temperatures of below 50°C and normal pressure, is performed.

## Revendications

1. Procédé pour la synthèse *in vitro* ou la synthèse *in vivo,* dans une cellule hôte transfectée isolée, de dioxyde de silicium, de silicones et d'autres composés de silicium (IV) ou de métal (IV) ainsi que de polymères mixtes de ces composés, un polypeptide selon SEQ ID N° 1 ou un complexe métallique de ce polypeptide étant utilisé pour la synthèse.

2. Procédé selon la revendication 1, **caractérisé en ce que**, pour la synthèse, des composés comme des acides siliciques, des monoalcoxysilane triols, des monoalcoxysilane diols, des monoalcoxysilanols, des dialcoxysilane diols, des dialcoxysilanols, des trialcoxysilanols, des tétraalcoxysilanes, des alkyl-, aryl- ou métallo-silane triols, des alkyl-, aryl- ou métallo-silane diols, des alkyl-, aryl- ou métallo-silanols, des alkyl-, aryl- ou métallo-monoalcoxysilane diols, des alkyl-, aryl- ou métallo-monoalcoxysilanols, des alkyl-, aryl- ou métallo-dialcoxysilanols, des alkyl-, aryl- ou métallo-trialcoxysilanes ou d'autres composés de métal (IV) sont utilisés en tant que réactifs (substrats).

3. Procédé de décomposition de dioxyde de silicium amorphe, de silicones et d'autres composés de silicium (IV) ou de métal (IV) ainsi que de polymères mixtes de ces composés, un polypeptide selon SEQ ID N° 1 ou un complexe métallique de ce polypeptide étant utilisé pour la décomposition.

4. Procédé de modification d'une structure ou d'une surface contenant de l'acide silicique ou un composé de silicium (IV) ou de métal (IV), un polypeptide selon SEQ ID N° 1 ou un complexe métallique de ce polypeptide étant utilisé pour la modification.

5. Polypeptide d'une silicatéine ß dérivée de *Suberites domuncula* selon SEQ ID N° 1 ou complexe métallique du polypeptide.

6. Acide nucléique, **caractérisé en ce qu'**il code pour un polypeptide selon la revendication 5.

7. Acide nucléique selon la revendication 6 sous la forme (a) d'un produit de recombinaison de protéine de fusion (protéine chimérique), (b) d'un produit de recombinaison ayant une expression protéique (site de clivage de la protéase) séparée ou (c) d'un produit de recombinaison ayant une expression protéique (expression des cassettes) séparée.

8. Procédé d'identification d'inhibiteurs ou d'activateurs d'un polypeptide de la silicatéine ß dérivée de *Suberites domuncula* selon SEQ ID N° 1, dans lequel a) un polypeptide de la silicatéine ß dérivée de *Suberites domuncula* selon SEQ ID N° 1 est fourni, b) le polypeptide issu de l'étape a) est mis en contact avec un inhibiteur ou un activateur potentiel, et c) la capacité du polypeptide à synthétiser ou à décomposer des silicates ou des silicones est mesurée.

9. Utilisation d'un polypeptide ou d'un acide nucléique selon l'une des revendications précédentes pour la résorption ou pour la modulation de la résorbabilité de silicones et d'implants en silicone.

10. Procédé selon la revendication 1, **caractérisé en ce que**, grâce à l'utilisation de silicatéine ß et a, une synthèse d'oligosilicates avec Siₙ(OH)₂ₙ₊₂ où n ≥ 2 en solution aqueuse et/ou en solution aqueuse / organique est réalisée à des températures inférieures à 50° C et à une pression normale.
